Europäisches Patentamt

European Patent Office

Office européen des brevets

(19)

(11) Numéro de publication : **0 197 808**
**B1**

(12) **FASCICULE DE BREVET EUROPÉEN**

(45) Date de publication du fascicule du brevet :
15.06.88

(51) Int. Cl.⁴ : **F 25 J   3/02, C 07 C   7/09**

(21) Numéro de dépôt : **86400448.6**

(22) Date de dépôt : **04.03.86**

(54) Procédé et installation de fractionnement de charges gazeuses.

(30) Priorité : **05.03.85 FR 8503222**

(43) Date de publication de la demande :
**15.10.86 Bulletin 86/42**

(45) Mention de la délivrance du brevet :
**15.06.88 Bulletin 88/24**

(84) Etats contractants désignés :
**BE DE GB IT NL SE**

(56) Documents cités :
**EP-A- 0 178 207**
**US-A- 4 251 249**
**US-A- 4 322 225**

(73) Titulaire : **COMPAGNIE FRANCAISE D'ETUDES ET DE CONSTRUCTION "TECHNIP"**
**170 Place Henri Regnault**
**F-92090 Paris la Défense (FR)**

(72) Inventeur : **Paradowski, Henri**
**32 Rue Serpente**
**F-95000 Cergy Pontoise (FR)**
Inventeur : **Parfait, Hervé**
**53 Rue Charlot**
**F-75003 Paris (FR)**
Inventeur : **Castel, Joelle**
**61 Rue du Val André**
**F-78560 Port Marly (FR)**
Inventeur : **Vasseur, Alain**
**61 Rue de Chazelles**
**F-75017 Paris (FR)**

(74) Mandataire : **Durand, Yves Armand Louis et al**
**Cabinet Z. Weinstein 20, Avenue de Friedland**
**F-75008 Paris (FR)**

## Description

La présente invention a essentiellement pour objet un procédé de fractionnement cryogénique de charges gazeuses quelconques et telles que par exemple les gaz naturels, les gaz associés à des condensats d'hydrocarbure, ou les gaz issus du traitement de fractions pétrolières.

L'invention vise également une installation pour la mise en œuvre de ce procédé.

On a déjà proposé des installations industrielles permettant d'extraire au choix soit l'éthane et les composés plus lourds que l'éthane, soit le propane et les composés plus lourds que le propane.

D'une manière générale, dans ces installations connues, la charge gazeuse est partiellement condensée par refroidissement à basse température puis séparée dans un ballon séparateur. Puis, la partie liquide est traitée dans une colonne de fractionnement classique, et on récupère en fond de cette colonne sous forme liquide le ou les composés lourds de la charge que l'on souhaite obtenir. Dans certains cas, on peut prévoir un cycle frigorifique pour assurer les besoins en froid de l'installation. Toutefois, ce genre d'installation ne permet pas d'extraire plus de 95 % du propane ou plus de 85 % de l'éthane contenu dans la charge, et ne présente donc pas un rendement excellent.

Par ailleurs, dans la demande de brevet EP-A-0 178 207 au nom de la demanderesse, on a proposé de remplacer le ballon séparateur des installations classiques par une colonne de purification-réfrigération par contact dans laquelle est introduite la charge gazeuse partiellement condensée pour y être traitée.

Cependant, une installation avec colonne de purification-réfrigération, telle que décrite dans la demande de brevet européen précitée, permet seulement d'extraire une seule coupe, par exemple le propane et les composés plus lourds que le propane (coupe $C_3+$), c'est-à-dire qu'elle ne permet pas d'extraire dans la même unité soit le propane et les composés plus lourds que le propane, soit l'éthane et les composés plus lourds que l'éthane.

Il s'agit donc là d'une lacune que la présente invention a pour but de combler en proposant un procédé et une installation qui présentent une grande souplesse d'emploi en raison du fait qu'avec ce procédé et cette installation, on peut immédiatement passer de l'extraction de la coupe $C_3+$ à l'extraction de la coupe $C_2+$ ou inversement, et qu'au surplus, on peut facilement atteindre des rendements de 99 % pour la coupe $C_3+$ et de 95 % pour la coupe $C_2+$.

A cet effet, l'invention a pour objet un procédé de fractionnement de charges gazeuses permettant d'extraire soit des composés plus lourds que le méthane (coupe $C_2+$) soit des composés plus lourds que l'éthane (coupe $C_3+$), et dans lequel on extrait ladite coupe $C_3+$ en injectant la charge gazeuse partiellement condensée en fond d'une colonne de purification-réfrigération par contact, laquelle colonne produit en tête du gaz résiduaire et en fond un liquide que l'on injecte dans une colonne de fractionnement pour obtenir au fond de cette colonne un liquide contenant ladite coupe $C_3+$ et en tête un distillat gazeux qui est condensé et injecté comme alimentation de tête de la colonne de purification-réfrigération, ce procédé étant caractérisé en ce que l'extraction de ladite coupe $C_3+$ est sélectivement permutable avec l'extraction de la coupe $C_2+$ que l'on peut obtenir :

- par recyclage d'une partie du gaz résiduaire comprimé qui est condensé dans un système d'échange puis injecté en tête de la colonne de purification-réfrigération,
- par injection du distillat gazeux issu de la colonne de fractionnement directement dans le fond de la colonne de purification-réfrigération et
- par injection du liquide issu du fond de la colonne de purification-réfrigération directement en tête de la colonne de fractionnement.

Ce procédé est encore caractérisé par le fait que, dans le cas où l'on veut extraire la coupe $C_2+$, la charge gazeuse, après condensation au moins partielle dans le système d'échange, est séparée d'une part en une phase liquide injectée au milieu de la colonne de fractionnement et d'autre part en une phase gazeuse qui est au moins partiellement condensée dans ledit système d'échange, puis injectée dans la colonne de purification-réfrigération, tandis que, dans le cas de l'extraction de la coupe $C_3+$, ladite charge gazeuse partiellement condensée dans le système d'échange réfrigéré est injectée directement et sans séparation dans la colonne de purification-réfrigération.

Un appoint en frigories dans le système d'échange sera réalisé grâce à un système de réfrigération qui, pour l'extraction de la coupe $C_2+$, fonctionne suivant un circuit frigorifique unique à plusieurs niveaux de pression, et qui, pour l'extraction de la coupe $C_2+$, fonctionne suivant deux circuits frigorifiques distincts et à savoir le circuit frigorifique unique précité et un second circuit frigorifique à un seul niveau de pression.

On précisera ici que, dans le circuit frigorifique unique fonctionnant pour extraire la coupe $C_3+$ ou pour extraire la coupe $C_2+$, on utilise de préférence un fluide composé d'un mélange d'hydrocarbures légers tels qu'éthane et propane, de poids moléculaire compris entre 30 et 40, ou bien d'un corps pur de type propane.

Dans le second circuit frigorifique précité qui fonctionne pour extraire la coupe $C_2+$, on utilisera de préférence un fluide composé d'un mélange d'hydrocarbures légers tels que méthane, éthylène, éthane ou propane, de poids moléculaire compris entre 26 et 36 ou un fluide composé d'un corps pur de type éthylène ou éthane.

Suivant une variante de réalisation de ce procédé, et dans le cas où l'on veut extraire la coupe $C_2+$, on dérive une partie de la charge gazeuse pour assurer le rebouillage de la colonne de fractionnement, on mélange la charge gazeuse dérivée avec la partie restante de la charge gazeuse préalablement et partiellement condensée dans le système d'échange, on sépare le mélange obtenu en une phase liquide et une phase gazeuse, et on sous-refroidit la phase liquide dans le système d'échange, tandis que la phase gazeuse est détendue puis mélangée à ladite phase liquide sous-refroidie pour constituer une phase mixte que l'on injecte dans la colonne de purification-réfrigération sous le lit de garnissage supérieur, alors que, dans le cas où l'on veut extraire la coupe $C_3+$, la charge gazeuse n'est pas dérivée mais condensée directement et partiellement dans le système d'échange, ce après quoi elle subit la séparation précitée pour former une phase gazeuse qui, après la détente précitée, est injectée directement dans la colonne de purification-réfrigération sous le lit de garnissage supérieur, et une phase liquide injectée directement dans ladite colonne de purification-réfrigération sous le lit de garnissage médian de cette colonne.

Suivant une autre caractéristique, en cas de besoin en frigories supplémentaires, une réfrigération par circuit frigorifique à plusieurs niveaux de pression par exemple est effectuée conjointement à la détente précitée.

Selon encore une autre caractéristique du procédé de l'invention, quelle que soit la coupe ($C_3+$ ou $C_2+$) que l'on souhaite extraire, la colonne de fractionnement précitée fonctionne à une pression supérieure à celle de la colonne de purification-réfrigération pour assurer l'injection du distillat issu de la colonne de fractionnement, condensé ou non, dans la colonne de purification-réfrigération.

L'invention vise également une installation pour la mise en œuvre du procédé ci-dessus et du type comprenant : une colonne de purification-réfrigération par contact associée à une colonne de fractionnement ; une conduite d'alimentation du liquide se trouvant en fond de la colonne de purification-réfrigération et reliant cette colonne à la colonne de fractionnement via un système d'échange ; et une conduite de reflux du distillat de la colonne de fractionnement reliant cette colonne à la colonne de purification-réfrigération via ledit système d'échange, cette installation étant essentiellement caractérisée en ce qu'elle comprend en outre :

- au moins une conduite de recirculation d'une partie du gaz résiduaire comprimé, laquelle conduite passe au travers du système d'échange et est raccordée à la tête de la colonne de purification-réfrigération,

- au moins un conduit permettant l'injection du distillat de la colonne de fractionnement directement et sans passer par le système d'échange, au fond de la colonne de purification-réfrigération, et

- au moins un conduit d'injection du liquide de la colonne de purification-réfrigération reliant directement et sans passer par le système d'échange, le fond de cette colonne à la tête de la colonne de fractionnement.

Suivant un mode de réalisation, la charge gazeuse condensée au moins partiellement dans le système d'échange est envoyée dans un séparateur comportant en fond une conduite reliée directement et de préférence au milieu de la colonne de fractionnement pour y injecter la phase liquide séparée, et en tête un conduit pour la phase gazeuse séparée qui traverse ledit système d'échange et auquel fait suite un conduit relié à la colonne de purification-réfrigération pour y injecter ladite phase gazeuse partiellement condensée, tandis qu'un moyen, tel que des vannes par exemple, est prévu pour court-circuiter ledit séparateur.

Suivant une autre caractéristique de cette installation, au système d'échange précité sont intégrés deux circuits frigorifiques fermés et indépendants fonctionnant respectivement à un seul niveau de pression et à un ou plusieurs niveaux de pression.

Selon un autre mode de réalisation, cette installation est caractérisée par une conduite de dérivation de la charge gazeuse, reliée à un système de rebouillage associé à la colonne de fractionnement et réunie avec une conduite dans laquelle passe la partie restante de la charge gazeuse, la réunion desdites conduites communiquant avec un séparateur dont le fond comporte une voie d'écoulement de la fraction liquide divisée en deux voies dont l'une est reliée audit système d'échange et dont l'autre est directement reliée à la colonne de purification-réfrigération, tandis que la tête du séparateur est reliée par une conduite à l'entrée d'un turbo-expandeur dont la sortie est raccordée à l'une des voies d'écoulement précitées pour ainsi constituer un mélange injecté par un conduit dans la colonne de purification-réfrigération à laquelle ledit conduit est relié.

On précisera ici qu'un circuit frigorifique par exemple du type à plusieurs niveaux de pression est associé à l'installation.

Suivant encore une autre caractéristique, le système d'échange est constitué par au moins un échangeur à plaques dans lequel les fluides qui se refroidissent sont descendants et les fluides qui se réchauffent sont ascendants.

A la colonne de purification-réfrigération est avantageusement associé un rebouilleur qui fait partie du système d'échange.

Selon encore une autre caractéristique de l'installation, la permutation de l'extraction de la coupe $C_3+$ avec l'extraction de la coupe $C_2+$ est tout simplement effectuée à l'aide de vannes à commande manuelle ou motorisée interposées sur les conduites précitées.

Mais d'autres caractéristiques et avantages de l'invention apparaîtront mieux dans la description détaillée qui suit et se réfère aux dessins annexés, donnés uniquement à titre d'exemple et dans lesquels :

La figure 1 est une vue schématique d'un exemple de réalisation d'une installation conforme aux principes de l'invention et utilisant un système de réfrigération à deux cycles frigorifiques.

La figure 2 est une vue schématique d'un autre exemple de réalisation d'une installation conforme à l'invention et utilisant un turbo-expandeur.

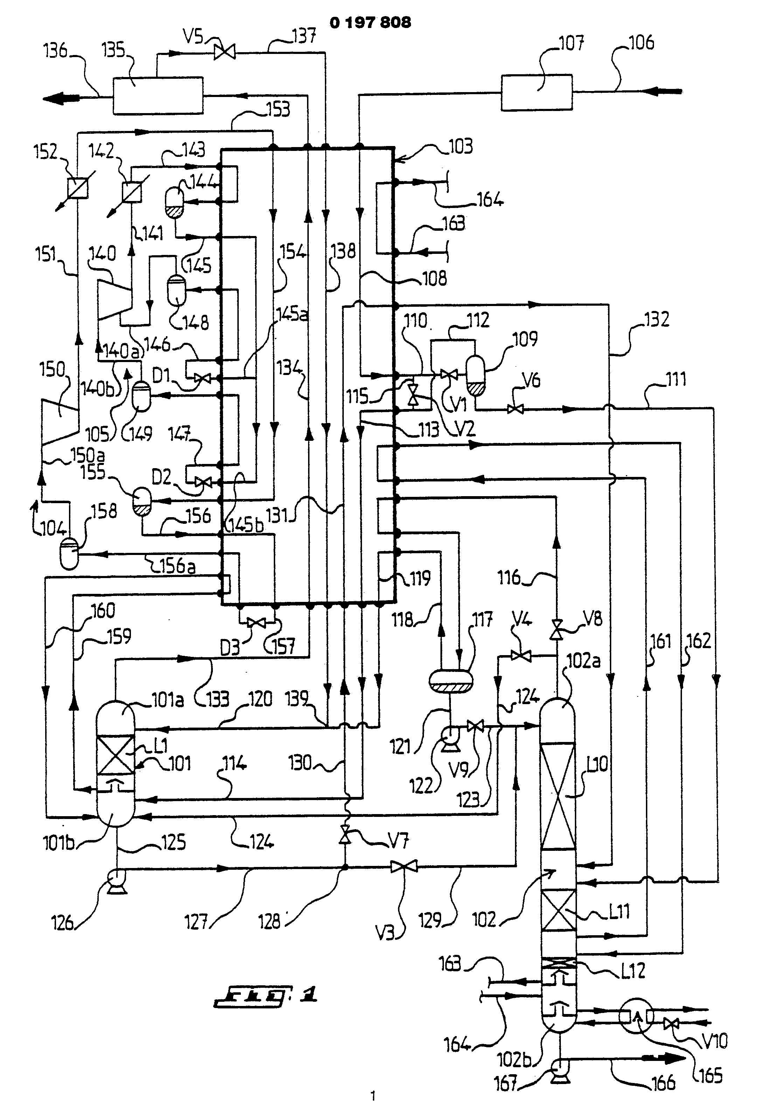

On se reportera tout d'abord à la figure 1 qui illustre le principe d'une unité d'extraction d'éthane et de composés plus lourds que l'éthane (coupe $C_2+$) ou de propane et de composés plus lourds que le propane (coupe $C_3+$), avec réfrigé-ration externe.

Cette installation comprend essentiellement une colonne 101 de purification-réfrigération par contact du type décrit dans la demande de brevet français précitée, une colonne de fractionnement 102 et un système d'échange 103 auquel sont associés deux circuits frigorifiques fermés et indépendants repérés d'une manière générale respectivement en 104 et 105.

On notera que la colonne 101 comporte un lit de garnissage supérieur $L_1$, tandis que la colonne de fractionnement 102 comporte au moins un lit de garnissage supérieur $L_{10}$, médian $L_{11}$ et inférieur $L_{12}$.

La charge gazeuse à traiter parvient à l'installation par une conduite 106 raccordée à une unité de séchage 107 à laquelle fait suite une voie de passage 108 traversant le système d'échange 103 et aboutissant à un séparateur 109 par une portion de conduite 110. Le fond du séparateur 109 est relié par une conduite 111 directement et de préférence au milieu de la colonne de fractionnement 102. La tête du séparateur est munie d'une conduite 112 dont une portion 113 traverse le système d'échange 103 et se prolonge par la portion de conduite visible en 114 et permettant l'injection de la phase gazeuse partiellement condensée en fond de la colonne de purification-réfrigération. Une vanne V1 est prévue sur la portion de conduite 110, et, comme on le verra plus loin, le séparateur 109 peut être court-cir-cuité par une conduite 115 reliant la portion de conduite 110 à la conduite 113 et équipée d'une vanne V2.

Le distillat gazeux en tête 102a de la colonne de fractionnement 102 est acheminé par une conduite 116 traversant le système d'échange 103 à un ballon de reflux 117. Ce ballon de reflux comporte en tête un conduit 118 d'alimentation en distillat gazeux qui est refroidi dans le système d'échange 103 en passant dans la portion de conduite 119 pour être condensé au moins partiellement et ainsi injecté par la portion de conduit 120 en tête 101a de la colonne de purification-réfrigération 101. Le ballon de reflux 117 comporte en fond un deuxième conduit 121 équipé d'une pompe 122 et assurant par la voie d'écoulement 123 le reflux en tête 102a de la colonne 102.

Un conduit 124 constituant une dérivation de la conduite 116 permet l'injection du distillat de la colonne de fractionnement 102 directement et sans passer par le système d'échange 103, dans

le fond 101b de la colonne de purification-réfrigération 101.

Le liquide en fond de cette colonne est récupéré dans une conduite 125 et envoyé par une pompe 126 dans la colonne de fractionnement 102 via 123.

Plus précisément, et comme on le voit bien sur la figure 1, le liquide sortant de la pompe 126 est acheminé à la colonne 102 par un conduit 127 qui se divise au point 128 en deux conduits et à savoir un conduit 129 relié directement et sans passer par le système d'échange 103 à la tête 102a de la colonne de fractionnement 102 via 123, et un conduit 130 comprenant une portion de conduit 131 traversant le système d'échange 103, et auquel fait suite une autre portion de conduit 132 reliée sensiblement au milieu de la colonne de fractionnement 102.

Le gaz résiduaire sortant en tête 101a de la colonne 101 passe dans une conduite 133 dont une portion 134 traverse le système d'échange 103 pour être ensuite raccordée à une unité de compression 135, ce après quoi le gaz résiduaire est évacué par une conduite 136. Une conduite 137 reliée à l'unité de compression 135 permet la recirculation du gaz résiduaire comprimé, cette conduite passant par une portion 138 au travers du système d'échange 103 et étant raccordée en 139 à la conduite 120 de reflux du distillat de la colonne de fractionnement 102 vers la tête de la colonne de purification-réfrigération 101.

Le circuit frigorifique fermé 105 comprend un compresseur 140 dont le refoulement est relié par une conduite 141 à un condenseur à réfrigérant d'origine externe. Ce condenseur 142 est relié par une conduite 143 traversant l'échangeur 103 à un ballon de recette 144. Le fond de ce ballon comporte une conduite 145 qui passe au travers de l'échangeur 103 pour sous-refroidir la phase liquide séparée. Cette conduite 145 se divise en deux voies 145a, 145b, chacune équipée d'une vanne de détente $D_1$, $D_2$. La phase liquide est ainsi vaporisée à deux niveaux de pression avant d'aboutir respectivement par les conduites 146, 147 traversant l'échangeur 103 à des ballons 148, 149 qui sont à leur tour respectivement reliés par les conduits 140a et 140b à l'étage intermédiaire et à l'étage basse pression du compresseur 140.

L'autre circuit frigorifique fermé 104 associé à l'échangeur 103 comprend essentiellement un compresseur 150 dont le refoulement est relié par une conduite 151 à un condenseur 152 à réfrigérant d'origine externe. La sortie de ce condenseur 152 est équipée d'une conduite 153 qui traverse par sa portion 154 l'échangeur 103 et aboutit à un ballon de recette 155. Le fond de ce ballon comprend une conduite 156 traversant l'échangeur 103, puis sortant de cet échangeur par une portion de conduite visible en 157 et munie d'une vanne de détente $D_3$. Ainsi la phase liquide sous-refroidie issue du ballon 155 est détendue et vaporisée dans la portion de conduite 156a pour parvenir à un ballon 158 raccordé par le conduit 150a à l'aspiration du compresseur 150.

Un rebouilleur 159, 160 est associé au fond de la colonne de purification-réfrigération 101, ce rebouilleur faisant partie du système d'échange 103, suivant l'exemple représenté sur la figure 1.

On a montré en 161 et 162 des conduites formant une boucle reliée à la colonne de fractionnement 102 et passant par le système d'échange 103 pour ainsi réaliser un rebouilleur intermédiaire susceptible de récupérer des frigories supplémentaires. Au fond 102b de la colonne de fractionnement est associé d'une part un rebouilleur constitué par des conduites 163 et 164 passant dans la section chaude du système d'échange 103, et d'autre part un autre rebouilleur à fluide chauffant externe repéré d'une manière générale en 165.

Le produit liquide recueilli en fond 102b de la colonne de fractionnement 102 est évacué par une conduite 166 via une pompe 167.

L'installation qui vient d'être décrite comprend en plus des vannes V1 et V2 susmentionnées d'autres vannes dont l'actionnement permettra de faire fonctionner l'installation en extraction de propane (coupe $C_3+$) ou en extraction d'éthane (coupe $C_2+$). C'est ainsi que l'on voit sur la figure 1 une vanne V3 sur le conduit 129, une vanne V4 sur la conduite 124, une vanne V5 sur la conduite 137, une vanne V6 sur la conduite 111, une vanne V7 sur le conduit 130, une vanne V8 sur la conduite 116, une vanne V9 sur la conduite 123 et une vanne V10 sur l'alimentation en fluide externe du rebouilleur 165.

On décrira dans ce qui suit le fonctionnement de l'installation de la figure 1 dans le cas d'une extraction de propane, puis dans le cas d'une extraction d'éthane, étant entendu qu'on n'insistera pas ici sur le rôle et le fonctionnement particulier de la colonne de purification-réfrigération, puisque cela a été décrit dans la demande de brevet français précité appartenant à la demanderesse.

Le fonctionnement de l'unité sera décrit en référence à un exemple calculé utilisant un gaz naturel dont la composition est la suivante :

- azote : 2,3 % molaire
- gaz carbonique : 0,3 % molaire
- méthane : 87,4 % molaire
- éthane : 7,0 % molaire
- propane : 2,1 % molaire
- butane : 0,9 % molaire.

Ce gaz est initialement à une pression de 30 bars et présente une température de 15 °C.

On décrira tout d'abord le fonctionnement de l'installation pour extraire le propane et les composés plus lourds que le propane ($C_3+$).

Dans ce cas de marche, les vannes V1, V3, V4, V5 et V6 sont fermées, et toutes les autres vannes sont ouvertes.

La charge gazeuse séchée est réfrigérée à haute pression dans le système d'échange 103 qui est constitué par un échangeur à plaques dans lequel les fluides qui se refroidissent sont descendants et les fluides qui se réchauffent sont ascendants. Plus précisément, le gaz séché passe dans les conduites 108 et 113 (vanne V1 fermée et vanne V2 ouverte), où il est refroidi jusqu'à

— 68 °C. Puis la charge gazeuse est introduite par la conduite 114 en fond 101b de la colonne de purification-réfrigération où elle contacte le reflux 120 constitué par le distillat gazeux 118 partiellement condensé en 119 à — 70 °C.

Les condensats recueillis en 125 en fond de la colonne de purification 101 sont pompés en 126 puis réchauffés via la canalisation 131 à 10 °C sous 30,3 bars avant d'alimenter la colonne de fractionnement 102 par la conduite 132.

La tête 102a de la colonne de fractionnement 102 comporte un système de condensation intégré au système d'échange 103 ou bien interne à ladite colonne, de sorte que les distillats qui en sortent par la conduite 116 sont condensés à — 31 °C, puis introduits dans le ballon 117 afin de produire le reflux gazeux passant dans la conduite 118, et le reflux liquide passant par la conduite 121, la pompe 122 et la conduite 123 pour ainsi retourner à la tête 102a de la colonne 102.

Le gaz résiduaire sortant de la colonne de purification-réfrigération 101 par la conduite 133, récupère des frigories en passant dans la conduite 134 traversant l'échangeur 103, pour être envoyé à 45 °C dans la section de compression 135, 136 où il est porté à une pression de 65 bars.

Le rebouillage de la colonne de fractionnement 102 est assuré par le fluide externe passant dans le rebouilleur 165, et la coupe $C_3+$ obtenue en fond 102b de la colonne 102 est disponible à une pression de 30,5 bars et à une température de 60 °C. On notera ici que dans le cas de l'extraction du propane qui est présentement décrite, les rebouilleurs 161, 162 d'une part et 163, 164 d'autre part ne sont pas utilisés. Il en est de même en ce qui concerne le séparateur 109 comme on l'a vu plus haut.

Quant à la réfrigération nécessaire au fonctionnement de l'installation, elle est assurée uniquement par le cycle frigorifique 105 utilisant de préférence un mélange réfrigérant d'éthane (47 % molaire) et de propane (53 % molaire).

Ce mélange, à la sortie du compresseur 140, se trouve à une pression de 30 bars et est partiellement condensé dans l'aéroréfrigérant 142 à 49 °C, puis est totalement condensé dans la partie supérieure chaude de l'échangeur 103 avant d'être introduit dans le ballon de recette 144. Le liquide issu de ce ballon est sous-refroidi dans l'échangeur 103 puis détendu et refroidi à deux niveaux de pression différents (flux 146 et 147), cela afin d'assurer la réfrigération nécessaire.

On décrira maintenant le fonctionnement de l'installation pour extraire l'éthane et les composés plus lourds que l'éthane (coupe $C_2+$).

Dans ce cas de figure, il convient simplement de fermer les vannes V2, V7, V8, V9 et V10, tandis que toutes les autres vannes V1, V3, V4, V5 et V6 sont ouvertes.

La charge gazeuse séchée en 107 est refroidie à — 60 °C à haute pression en passant par la conduite 108 dans l'échangeur à plaques 103, puis ladite charge est introduite dans le ballon séparateur 109 (vanne V1 ouverte et vanne V2 fermée). La phase liquide 111 est directement injectée dans la colonne 102. Par contre, la phase vapeur 112 passant dans la conduite 113 est refroidie à — 91,7 °C et introduite par la conduite 114 en fond de la colonne de purification-réfrigération 101 où elle contacte le fluide réfrigérant au moins partiellement condensé 120 qui, comme on l'a vu plus haut, est constitué par le recyclage à haute pression (43,7 bars) d'une fraction 137 du gaz résiduaire prélevé sur l'étage intermédiaire de la section de compression 135, 136 laquelle fraction est totalement condensée en 138 dans l'échangeur 103 avant d'aboutir à la colonne 101 par la conduite 120.

Ici, le flux en fond de la colonne 101 est réchauffé et partiellement vaporisé dans le système d'échange 103 (conduites 159, 160), afin d'assurer le rebouillage de ladite colonne 101.

Le liquide du fond de cette colonne est envoyé par la pompe 126 directement en tête 102a de la colonne de fractionnement 102, via les conduits 127 et 129 (vanne V3 ouverte, vanne V7 fermée).

Le rebouilleur intermédiaire 161, 162 permet de récupérer des frigories supplémentaires, tandis que le rebouillage 163, 164 de la colonne 102 est assuré dans la partie supérieure chaude de l'échangeur 103, jusqu'à 20 °C.

La coupe $C_2+$ est recueillie en 166 à une température de 20 °C sous 30 bars, et la tête 102a de la colonne 102 alimente directement le fond de la colonne de purification-réfrigération 101 grâce à la conduite 124 (vanne V4 ouverte et vanne V8 fermée).

Le gaz résiduaire sortant de la colonne 101 par la conduite 133 récupère des frigories dans l'échangeur 103 par passage dans la conduite 134 et est envoyé à 40 °C dans la section de compression 135, 136 pour être porté à une pression de 65 bars.

La réfrigération nécessaire au fonctionnement de l'unité pour extraire la coupe $C_2+$ est apportée par le cycle frigorifique 105, et aussi par le cycle frigorifique 104.

Le cycle frigorifique 104 utilise de préférence un mélange réfrigérant d'éthylène (99 % molaire) et de propane (1 % molaire).

Au refoulement du compresseur 150, ce mélange réfrigérant se trouve à une pression de 13 bars et est refroidi à 49 °C dans l'aéroréfrigérant 152. Puis, grâce au cycle frigorifique 105, le mélange réfrigérant précité est totalement condensé lorsqu'il parvient au ballon de recette 155. Le liquide 156 issu de ce ballon est ensuite sous-refroidi à — 94 °C en passant dans l'échangeur 103, puis détendu à 1,6 bar ($D_3$) et vaporisé jusqu'à — 65 °C afin d'assurer la réfrigération nécessaire de la partie inférieure et froide de l'échangeur 103.

Quant au cycle frigorifique 105 il fonctionne exactement comme cela a été décrit précédemment à propos du fonctionnement de l'installation dans le cas où l'on veut extraire la coupe $C_3+$ ; toutefois, dans le cas du fonctionnement de l'installation pour extraire la coupe $C_2+$, le mélange réfrigérant circulant dans le circuit 105 est de préférence constitué par un mélange d'éthane (50 % molaire) et de propane (50 % molaire).

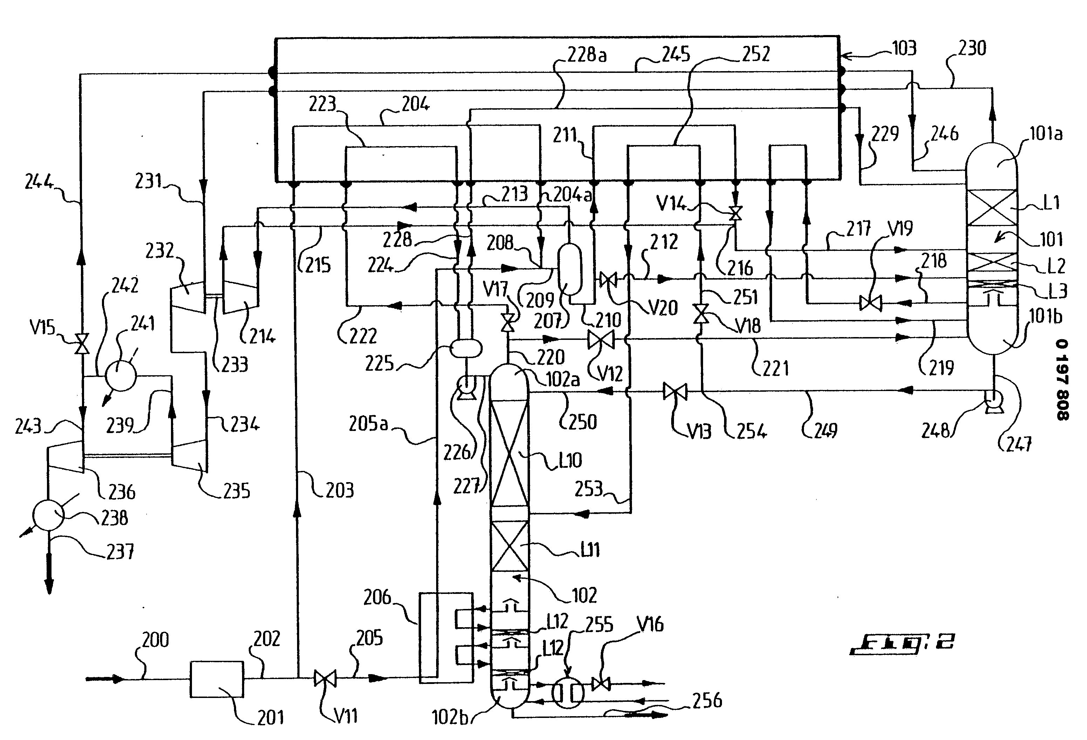

On se reportera maintenant à la figure 2 pour décrire la structure et le fonctionnement d'un deuxième mode de réalisation d'installation conforme aux principes de l'invention et utilisant au moins un turbo-expandeur.

Comme dans le mode de réalisation décrit précédemment, cette installation comprend essentiellement une colonne 101 de purification-réfrigération par contact, une colonne de fractionnement 102 et un système d'échange 103. On notera que la colonne 101 comporte un lit de garnissage supérieur $L_1$ et au moins un lit de garnissage médian $L_2$ et inférieur $L_3$, tandis que la colonne de fractionnement 102 comporte au moins un lit de garnissage supérieur $L_{10}$, médian $L_{11}$ et inférieur $L_{12}$. Cette installation, comme celle représentée sur la figure 1 permet à elle seule, à l'aide de réglages simples, de passer d'un type d'extraction à un autre, c'est-à-dire de l'extraction d'une coupe $C_3+$ à l'extraction d'une coupe $C_2+$.

La charge gazeuse parvient à l'installation par une conduite 200 raccordée à une unité de séchage 201 équipée d'une voie de sortie 202 du gaz séché, laquelle voie se divise en deux conduites et à savoir : une conduite 203 à laquelle fait suite une portion 204 du système d'échange 103 elle-même suivie par une portion de conduite 204a ; et une conduite de dérivation 205 traversant un système de rebouillage 206 associé à la colonne de fractionnement 102, ladite conduite 205 se prolongeant par une portion 205a qui aboutit à un séparateur de phases 207. La portion de conduite 204a est reliée en 208 à la portion de conduite 205a en amont du séparateur 207 pour former une conduite unique 209 communiquant avec ledit séparateur.

Le fond du séparateur 207, comporte une voie d'écoulement 210 qui se divise en deux voies et à savoir une voie 211 traversant le système d'échange 103 et une voie 212 directement reliée à la colonne de purification-réfrigération 101.

La tête du séparateur 207 est reliée par une conduite 213 à l'entrée d'un turbo-expandeur 214 dont la sortie est raccordée par une conduite 215 à la voie d'écoulement 211 mentionnée précédemment. Plus précisément, la voie 211 sortant du système d'échange 103 est reliée à la conduite 215 au point 216 pour ainsi réaliser un mélange injecté par un conduit unique 217 dans la colonne 101 de purification-réfrigération par contact. Cette colonne est équipée d'un système de rebouillage formé par les conduites 218, 219 formant une boucle passant par le système d'échange 103.

La tête 102a de la colonne de fractionnement 102 comporte une voie 220 d'écoulement du distillat, laquelle voie se divise en deux conduites, à savoir une conduite 221 qui ne passe pas par le système d'échange 103 et qui permet l'injection du distillat directement en fond 101b de la colonne de purification-réfrigération 101, et une conduite 222 reliée à la portion 223 du système d'échange 103.

La portion 223 dudit système d'échange est reliée par une conduite 224 à un ballon de reflux 225. Le fond de ce ballon de reflux est relié par une conduite 227 via une pompe 226 à la tête 102a de la colonne 102 dans laquelle est ainsi injectée la phase liquide du ballon 225.

La phase gazeuse du ballon 225 est acheminée par une conduite 228 à une portion 228a du système d'échange 103, puis elle est injectée par une conduite 229 en tête 101a de la colonne de purification-réfrigération 101.

La tête 101a de cette colonne est reliée à une conduite 230 qui assure le passage du gaz résiduaire au travers de l'échangeur 103, puis, à la sortie de l'échangeur 103, la conduite 231 est raccordée à l'aspiration d'un compresseur 232 attelé au turbo-expandeur 214, comme on l'a montré schématiquement en 233.

Le refoulement du compresseur 232 est relié par une conduite 234 à l'aspiration du premier étage 235 de la section de compression, puis transféré par un conduit 239 via un échangeur 241 au deuxième étage 236 de la section de compression, le gaz résiduaire étant évacué par une conduite 237 munie d'un système d'échangeur-réfrigérant final 238. La conduite de sortie 242 de l'échangeur 241 se divise en deux branches et à savoir une conduite 243 reliée à l'aspiration du deuxième étage de compression 236 et une conduite 244 de recyclage du gaz résiduaire, à laquelle fait suite une portion 245 du système d'échange 103, elle-même suivie par une conduite 246 raccordée à la tête 101a de la colonne de purification-réfrigération 101.

Le liquide en fond 101b de cette colonne est recueilli dans une conduite 247 munie d'une pompe 248 dont la sortie est équipée d'un conduit 249 qui se divise en deux voies d'écoulement et à savoir un conduit 250 directement relié à la tête de la colonne de fractionnement 102 et un conduit 251 suivi d'une portion 252 du système d'échange 103 et à laquelle fait suite une conduite 253 reliée sensiblement au milieu de ladite colonne 102. On a montré en 254 le point de réunion des conduits 250 d'une part et 251, 252, 253 d'autre part.

Un rebouilleur 255 est associé au fond 102b de la colonne 102, et les composés lourds recueillis en fond de cette colonne sont évacués par une conduite 256.

L'installation qui vient d'être décrite comprend une pluralité de vannes à commande manuelle ou motorisée, qui permettent le fonctionnement de l'installation de façon à extraire soit la coupe $C_3+$ soit la coupe $C_2+$. C'est ainsi que l'on voit sur la figure 2 une vanne V11 sur la conduite de dérivation 205, une vanne V12 sur le conduit 221, une vanne V13 sur le conduit 250, une vanne V14 sur la conduite 211, une vanne V15 sur la conduite 244, une vanne V16 sur la voie de passage du fluide externe dans le rebouilleur 255, une vanne V17 sur la conduite 222, une vanne V18 sur le conduit 251, une vanne V19 sur le rebouilleur 218-219, et une vanne V20 sur la conduite 212.

On décrira dans ce qui suit le fonctionnement de cette installation en extraction de propane et en extraction d'éthane. Ce fonctionnement sera décrit en référence à un exemple calculé utilisant un gaz naturel présentant la composition suivante :

- azote : 2,3 % molaire
- gaz carbonique : 0,3 % molaire
- méthane : 87,4 % molaire
- éthane : 7,0 % molaire
- propane : 2,1 % molaire
- butane : 0,9 % molaire.

Ce gaz est introduit dans l'installation par la conduite 200 à une température de 15 °C sous une pression de 50 bars.

Pour extraire le propane et les composés plus lourds que le propane (coupe $C_3+$), on ferme les vannes V11, V12, V13, V14 et V15, tandis que les vannes V16, V17, V18, V19 et V20 sont ouvertes.

La charge gazeuse séchée à 15 °C et haute pression en 201 s'écoule dans les conduites 202 et 203 puis est partiellement condensée en 204 dans l'échangeur à plaques 103 jusqu'à une température de — 40 °C, ce après quoi elle est introduite dans le ballon séparateur 207. La phase liquide issue de ce ballon s'écoule dans la conduite 212 où elle est détendue à 20 bars et alimente la colonne de purification-réfrigération 101 sous le lit de garnissage médian $L_2$.

La phase vapeur 213 issue du ballon 207 est détendue dans le turbo-expandeur 214 jusqu'à 20 bars pour atteindre une température de — 76 °C. Elle est ensuite injectée dans la colonne 101 par les conduites 215 et 217, ladite colonne 101 étant rebouillie dans la section froide de l'échangeur 103 (flux 218, 219) de — 73 à — 60 °C.

Dans cette colonne 101 la charge gazeuse contacte le reflux 229 injecté en tête 101a, ce reflux étant constitué par le distillat gazeux issu du ballon 225 et totalement condensé à — 78 °C dans la conduite 228 traversant l'échangeur 103.

Le gaz résiduaire issu de la colonne 101 et sortant par la conduite 230 est réchauffé dans l'échangeur 103 jusqu'à atteindre une température de 41,5 °C, et il est envoyé ensuite par la conduite 231 dans le compresseur 232 attelé au turbo-expandeur 214. Le gaz résiduaire passant ensuite dans la conduite 234 est comprimé en deux étages 235 et 236 de la section de compression jusqu'à une pression de 66,4 bars, tandis que l'échangeur-réfrigérant intermédiaire 241 permet de ramener le gaz passant dans la conduite 239 de 101 °C à 49 °C. Le gaz résiduaire est refroidi dans l'échangeur-réfrigérant final 238 avant d'être évacué à une pression de 66 bars.

Les condensats en fond de la colonne de purification-réfrigération 101 sont refoulés par la pompe 248 dans le conduit 249 à une pression de 22 bars, puis acheminés par la conduite 251, dans la portion 252 de l'échangeur 103, pour y être réchauffés jusqu'à une température de 13,5 °C, avant d'alimenter par 253, la colonne de fractionnement 102 sous le lit de garnissage supérieur L₁₀.

Le distillat en tête 102a de la colonne 102 est envoyé via les conduites 220, 222 dans l'échangeur 103, où il est au moins partiellement condensé en 223 à — 23,5 °C, puis introduit par la conduite 224 dans le ballon 225 afin de produire un reflux liquide 227 injecté en tête de la colonne 102 et un distillat gazeux envoyé en tête 101a de la colonne 101 par la conduite 229, après condensation totale dans la portion 228a de l'échangeur 103, comme expliqué précédemment.

On notera ici qu'il est possible, sans sortir du cadre de l'invention, d'intégrer la condensation de la colonne 102 dans la tête de cette colonne, ce qui permettra évidemment de supprimer le ballon 225 et la pompe de reflux 226. Dans ce cas là, les frigories nécessaires à la condensation seront apportées par un fluide froid issu de l'échangeur à plaques 103.

Enfin, le rebouillage de la colonne 102 sera assuré par un fluide externe passant dans le rebouilleur 255 (vanne V16 ouverte), et la coupe $C_3+$ recueillie dans la conduite 256 sera à une température de 75,6 °C, et à une pression de 22 bars.

On décrira maintenant le fonctionnement de l'installation représentée sur la figure 2, lorsqu'on veut obtenir l'éthane et les composés plus lourds que l'éthane (coupe $C_2+$).

Dans ce cas, les vannes V11, V12, V13, V14 et V15 sont ouvertes, alors que les vannes V16, V17, V18, V19 et V20 sont fermées.

La charge gazeuse séchée en 201 à haute pression et à 15 °C se sépare en deux flux 203 et 205. Le flux 205 est partiellement condensé en 205a à — 62 °C en assurant le rebouillage de la colonne de fractionnement 102 dans l'échangeur 206. Quant au flux 203, il est lui aussi partiellement condensé à — 62 °C dans la portion 204 de l'échangeur 103. Les flux 205a et 204a sont mélangés en 208, avant d'entrer dans le ballon séparateur 207.

La phase liquide 210 issue de ce séparateur est sous-refroidie en 211 jusqu'à — 107 °C dans l'échangeur à plaques 103, puis détendue à 13,8 bars.

La phase vapeur 213 issue du séparateur 207 est détendue dans le turbo-expandeur 214 jusqu'à 13,8 bars et atteint une température de — 107 °C pour ensuite passer dans la conduite 215 afin d'être mélangée en 216 au flux 211 pour finalement alimenter la colonne de purification 101 par la conduite 217 entre les lits de garnissage supérieur L₁ et médian L₂.

Dans la colonne 101, la fraction gazeuse du flux 217 contacte le flux 246 qui est constitué par le recyclage à haute pression (33,8 bars) d'une fraction 244 du gaz résiduaire totalement condensé en 245 dans l'échangeur 103, ladite fraction étant prélevée sur l'étage intermédiaire de la section de compression 235, 236.

Le gaz résiduaire sortant de la colonne 101 par la conduite 230 est réchauffé dans l'échangeur 103 jusqu'à atteindre une température de 15 °C, et est envoyé par la conduite 231 à l'aspiration du compresseur 232 de façon à être porté d'une pression de 12,6 à 16,2 bars. Le refoulement 234 du compresseur 232 est ensuite comprimé à 67 bars dans les deux étages de compression 235 et 236.

L'échangeur-réfrigérant 241 permet de réfrigérer le gaz 239 de 116 °C à 49 °C, tandis que le gaz résiduaire recyclé vers la tête 101a de la colonne 101 est prélevé à la sortie de cet échangeur. Le gaz résiduaire refoulé par le deuxième étage de compression 236 est refroidi à 49 °C dans l'échangeur final 238 et est disponible à une pression de 66,4 bars.

Les condensats issus du fond de la colonne de purification-réfrigération 101 sont refoulés par la pompe 248 pour alimenter directement via les conduits 249 et 250 la tête de la colonne de fractionnement 102, tandis que les distillats en tête de cette colonne sont à leur tour directement envoyés dans le fond de la colonne de purification 101 par les conduits 220, 221.

Enfin, on recueillera la coupe $C_2+$ dans la conduite 256 prévue en fond de la colonne de fractionnement 102.

On a donc réalisé suivant l'invention un procédé et une installation d'extraction cryogénique permettant, dans la même unité, d'extraire soit la coupe $C_2+$ soit la coupe $C_3+$ avec des rendements très élevés pouvant atteindre 99 % pour la coupe $C_3+$ et 95 % pour la coupe $C_2+$, étant entendu que l'on peut passer très facilement et rapidement d'un type d'extraction à un autre en effectuant des réglages très simples.

Bien entendu, l'invention n'est pas limitée aux modes de réalisation décrits et illustrés qui n'ont été donnés qu'à titre d'exemple.

C'est ainsi que l'on pourrait parfaitement, sans sortir du cadre de l'invention, associer au turbo-expandeur de la variante visible sur la figure 2, un circuit frigorifique supplémentaire du type à plusieurs niveaux de pression par exemple.

**Revendications**

1. Procédé de fractionnement de charges gazeuses permettant d'extraire soit des composés plus lourds que le méthane (coupe $C_2+$) soit des composés plus lourds que l'éthane (coupe $C_3+$), et dans lequel on extrait ladite coupe $C_3+$ en injectant la charge gazeuse partiellement condensée en fond d'une colonne de purification-réfrigération (101) par contact, laquelle colonne produit en tête du gaz résiduaire et en fond un liquide que l'on injecte dans une colonne de fractionnement (102) pour obtenir au fond de cette colonne un liquide contenant ladite coupe $C_3+$ et en tête un distillat gazeux qui est condensé et injecté comme alimentation de tête de la colonne de purification-réfrigération, caractérisé en ce que l'extraction de ladite coupe $C_3+$ est sélectivement permutable avec l'extraction de la coupe $C_2+$ que l'on peut obtenir :

- par recyclage d'une partie du gaz résiduaire comprimé (137, 244) qui est condensé (138, 245) dans un système d'échange (103) puis injecté (120, 246) en tête de la colonne de purification-réfrigération (101),

- par injection (124 ; 220, 221) du distillat gazeux issu de la colonne de fractionnement (102) directement dans le fond de la colonne de purification-réfrigération (101),

- et par injection (125, 127, 129 ; 247, 249, 250) du liquide issu du fond de la colonne de purification-réfrigération (101) directement en tête de la colonne de fractionnement (102).

2. Procédé selon la revendication 1, caractérisé en ce que, dans le cas où l'on veut extraire la coupe $C_2+$, la charge gazeuse (106), après condensation au moins partielle (108) dans le système d'échange (103), est séparée (109) d'une part en une phase liquide (111) injectée au milieu de la colonne de fractionnement (102) et d'autre part en une phase gazeuse (112) qui est au moins partiellement condensée (113) dans ledit système d'échange puis injectée (114) dans la colonne de purification-réfrigération (101), tandis que, dans le cas de l'extraction de la coupe $C_3+$, ladite charge gazeuse (106) partiellement condensée (108, 113) dans le système d'échange réfrigéré est injectée (114) directement et sans séparation dans la colonne de purification-réfrigération (figure 1).

3. Procédé selon la revendication 1 ou 2, caractérisé en ce qu'on réalise un appoint en frigories dans le système d'échange précité grâce à un système de réfrigération qui, pour l'extraction de la coupe $C_3+$, fonctionne suivant un circuit frigorifique unique (105) à plusieurs niveaux de pression et qui, pour l'extraction de la coupe $C_2+$, fonctionne suivant deux circuits frigorifiques distincts et à savoir le circuit frigorifique unique précité (105) et un second circuit frigorifique (104) à un seul niveau de pression.

4. Procédé selon la revendication 3, caractérisé en ce que, dans le circuit frigorifique unique précité (105) qui fonctionne pour extraire la coupe $C_3+$ ou pour extraire la coupe $C_2+$, on utilise de préférence un fluide composé d'un mélange d'hydrocarbures légers tels qu'éthane et propane, de poids moléculaire compris entre 30 et 40, ou bien d'un corps pur de type propane.

5. Procédé selon la revendication 3, caractérisé en ce que dans le second circuit frigorifique précité (104) qui fonctionne pour extraire la coupe $C_2+$, on utilise de préférence un fluide composé d'un mélange d'hydrocarbures légers tels que méthane, éthylène, éthane ou propane, de poids moléculaire compris entre 26 et 36, ou composé d'un corps pur de type éthylène ou éthane.

6. Procédé suivant la revendication 1, caractérisé en ce que, dans le cas où on veut extraire la coupe $C_2+$, on dérive (205, 205a) une partie de la charge gazeuse (200, 202) pour assurer le rebouillage (206) de la colonne de fractionnement (102), on mélange (208) la charge gazeuse dérivée avec la partie restante (203) de la charge gazeuse préalablement et partiellement condensée (204) dans le système d'échange (103), on sépare (207) le mélange obtenu en une phase liquide et une phase gazeuse, et on sous-refroidit (211) la phase liquide dans le système d'échange (103), tandis que la phase gazeuse (213) est détendue (214) puis mélangée (216) à ladite phase liquide sous-refroidie (211) pour constituer une phase mixte que l'on injecte (217) dans la colonne de purification-réfrigération (101) sous le lit de garnissage supérieur ($L_1$), alors que, dans le cas où l'on veut extraire la coupe $C_3+$, la charge gazeuse (200) n'est pas dérivée mais condensée directement et partiellement (204) dans le système d'échange (103), ce après quoi elle subit la séparation précitée (207) pour former une phase gazeuse (213) qui, après la détente précitée (214), est injectée directement (215, 217) dans la colonne (101) sous le lit de garnissage supérieur, et une phase liquide (210, 212) injectée directement dans la colonne de purification-réfrigération (101) sous le lit de garnissage médian ($L_2$) (figure 2).

7. Procédé selon la revendication 6, caractérisé en ce que, en cas de besoin en frigories supplémentaires, une réfrigération par circuit frigorifique à plusieurs niveaux de pression par exemple est effectuée conjointement à la détente précitée (214).

8. Procédé selon l'une des revendications 1 à 7, caractérisé en ce que, quelle que soit la coupe ($C_3+$ ou $C_2+$) que l'on souhaite extraire, la colonne de fractionnement précitée (102) fonctionne à une pression supérieure à celle de la colonne de purification-réfrigération (101) pour assurer l'injection du distillat issu de la colonne de fractionnement, condensé ou non, dans la colonne de purification-réfrigération.

9. Installation pour la mise en œuvre du procédé selon l'une des revendications précédentes et du type comprenant : une colonne de purification-réfrigération par contact (101) associée à une colonne de fractionnement (102) ; au moins une conduite (125, 127, 131, 132 ; 247, 249, 251, 252, 253) d'alimentation du liquide se trouvant en fond de la colonne de purification-réfrigération (101) et reliant cette colonne à la colonne de fractionnement (102) via un système d'échange (103) ; et au moins une conduite (118, 119, 120 ; 228, 229) de reflux du distillat de la colonne de fractionnement (102) reliant cette colonne à la colonne de purification-réfrigération (101) via ledit système d'échange, caractérisée en ce qu'elle comprend en outre :

- au moins une conduite (137, 138, 120 ; 244, 245, 246) de recirculation d'une partie du gaz résiduaire comprimé, laquelle conduite passe au travers dudit système d'échange (103) et est raccordée à la tête (101a) de la colonne de purification-réfrigération,

- au moins un conduit (124 ; 220, 221) permettant l'injection du distillat (102a) de la colonne de fractionnement directement et sans passer par le système d'échange (103) au fond (101b) de la colonne de purification-réfrigération (101), et

- au moins un conduit (125, 127, 129 ; 247, 249, 250) d'injection du liquide de la colonne de purification-réfrigération reliant directement et sans passer par le système d'échange, le fond (101b) de cette colonne à la tête (102a) de la colonne de fractionnement (102).

10. Installation selon la revendication 9, caractérisée en ce que la charge gazeuse (106) condensée au moins partiellement (108) dans le système d'échange (103) est envoyée (110) dans un séparateur (109) comportant en fond une conduite (111) reliée directement et de préférence au milieu de la colonne de fractionnement (102) pour y injecter la phase liquide séparée, et en tête un conduit (112) pour la phase gazeuse séparée (113) qui traverse ledit système d'échange (103), et auquel fait suite un conduit (114) relié à la colonne de purification-réfrigération pour y injecter ladite phase gazeuse partiellement condensée, tandis qu'un moyen, tel que des vannes (V1, V2), est prévu pour court-circuiter ledit séparateur (figure 1).

11. Installation suivant la revendication 9 ou 10, caractérisée en ce qu'audit système d'échange (103) sont intégrés deux circuits frigorifiques fermés et indépendants (105, 104) fonctionnant respectivement à un seul niveau de pression et à un ou plusieurs niveaux de pression (figure 1).

12. Installation suivant la revendication 9, caractérisée par une conduite de dérivation (205, 205a) de la charge gazeuse, reliée à un système de rebouillage (206) associé à la colonne de fractionnement (102) et réunie avec une conduite (203, 204a) dans laquelle passe la partie restante de la charge gazeuse, la réunion (208) desdites conduites communiquant avec un séparateur (207) dont le fond comporte une voie d'écoulement (210) de la fraction liquide divisée en deux voies dont l'une (211) est reliée audit système d'échange (103) et dont l'autre (212) est directement reliée à la colonne de purification-réfrigération (101), tandis que la tête du séparateur (207) est reliée par une conduite (213) à l'entrée d'un turbo-expandeur (214) dont la sortie est raccordée à l'une (211) des voies d'écoulement précitées pour ainsi constituer un mélange injecté par un conduit (217) dans la colonne de purification-réfrigération (101) à laquelle il est relié (figure 2).

13. Installation suivant la revendication 12, caractérisée en ce qu'un circuit frigorifique par exemple du type à plusieurs niveaux de pression est associé à ladite installation.

14. Installation suivant l'une des revendications 9 à 13, caractérisée en ce que le système d'échange précité est constitué par au moins un échangeur à plaques dans lequel les fluides qui se refroidissent sont descendants et les fluides qui se réchauffent sont ascendants.

15. Installation selon l'une des revendications 9 à 14, caractérisée par un rebouilleur (159, 160 ; 218, 219) associé à la colonne de purification-réfrigération (101) et faisant partie du système d'échange (103).

16. Installation suivant l'une des revendications 9 à 15, caractérisée en ce que la permutation de l'extraction de la coupe $C_3+$ avec l'extraction de la coupe $C_2+$ est effectuée à l'aide de vannes (V1 à V20) à commande manuelle ou motorisée interposées sur les conduites précitées.

**Claims**

1. A process of fractionating gas feeds for extracting therefrom either compounds heavier than methane ($C_2+$ cut) or compounds heavier than ethane ($C_3+$ cut), wherein the said $C_3+$ cut is extracted by injecting the partially condensed gas feed into the bottom of a contact purifying-refrigerating column (101), which column produces residual gas at its top and a liquid at its bottom, which liquid is injected into a fractionating column (102) to obtain at the bottom of the said fractionating column a liquid containing the said $C_3+$ cut, and at its top, a gaseous distillate which is condensed and injected as top supply into the purifying-refrigerating column, characterized in that the extraction of the said $C_3+$ cut is selectively permutable with the extraction of the $C_2+$ cut which can be obtained
   - by recirculating a portion of the compressed (137, 244) residual gas which is condensed (138, 245) in an exchange system (103) and then injected (120, 246) into the top of the purifying-refrigerating column (101),
   - by injecting (124 ; 220, 221) the gaseous distillate leaving the fractionating column (102) directly into the bottom of the purifying-refrigerating column (101),
   - and by injecting (125, 127, 129 ; 247, 249, 250) the liquid leaving the bottom of the purifying-refrigerating column (101) directly into the top of the fractionating column (102).

2. A process according to claim 1, characterized in that, where the cut to be extracted is $C_2+$, the gas feed (106), after being condensed at least partially (108) in the exchange system (103), is separated (109), on the one hand, into a liquid phase (111) which is injected into the middle of the fractionating column (102) and, on the other hand, into a gaseous phase (112) which is at least partially condensed (113) in the said exchange system and then injected (114) into the purifying-refrigerating column (101), whereas, if the cut to be extracted is $C_3+$, the said gas feed (106) partially condensed (108, 113) in the refrigerated exchange system is injected (114) directly and without separation into the purifying-refrigerating column (Figure 1).

3. A process according to claim 1 or 2, characterized in that additional cold is created in the said exchange system owing to a refrigerating system which, if the cut to be extracted is $C_3+$, operates as a single refrigerating circuit (105) with several pressure levels, and which, if the cut to be extracted is $C_2+$, operates as two distinct refrigerating circuits, namely the said single refrigerating circuit (105) and a second refrigerating circuit (104) with a single pressure level.

4. A process according to claim 3, characterized in that, in the said single refrigerating circuit (105) operating for the extraction of the $C_3+$ cut or for the extraction of the $C_2+$ cut, use

is preferably made of a medium consisting of a mixture of light hydrocarbons such as ethane and propane, of a molecular weight ranging from 30 to 40, or of a pure substance of the propane type.

5. A process according to claim 3, characterized in that in the said second refrigerating circuit (104) operating for the extraction of the $C_2+$ cut, use is preferably made of a medium consisting of a mixture of light hydrocarbons such as methane, ethylene, ethane or propane, of a molecular weight ranging from 26 and 36, or consisting of a pure substance of the ethylene or ethane type.

6. A process according to claim 1, characterized in that, where the cut to be extracted is $C_2+$, a portion of the gas feed (200, 202) is derived (205, 205a) for the purpose of reboiling (206) the fractionating column (102), the derived gas feed is mixed (208) with the remainder (203) of the gas feed previously and partially condensed (204) in the exchange system (103), the mixture obtained is separated (207) into a liquid phase and a gaseous phase, and the liquid phase is subcooled (211) in the exchange system (103), whereas the gaseous phase (213) is expanded (214) and then mixed (216) with the said subcooled liquid phase (211) to form a combined phase which is injected (217) into the purifying-refrigerating column (101) under the upper packing layer ($L_1$), whereas, if the cut to be extracted is $C_3+$, the gas feed (200) is not derived but condensed directly and partially (204) in the exchange system (103), whereafter it is subjected to the said separation (207) to form a gaseous phase (213) which, after the said expansion (214), is injected directly (215, 217) into the column (101) under the upper packing layer, and a liquid phase (210, 212) which is injected directly into the purifying-refrigerating column (101) under the middle packing layer ($L_2$) (Figure 2).

7. A process according to claim 6, characterized in that, in case additional cold is needed, a refrigeration through a refrigerating circuit with several pressure levels, for example, is performed jointly with the aforesaid expansion (214).

8. A process according to one of claims 1 to 7, characterized in that, irrespective of the cut ($C_3+$ or $C_2+$) which it is desired to extract, the said fractionating column (102) operates at a pressure higher than that of the purifying-refrigerating column (101) so as to ensure the injection of the distillate issued from the fractionating column, and either condensed or not, into the purifying-refrigerating column.

9. An apparatus for carrying out the process according to one of the foregoing claims, of the type including : a contact purifying-refrigerating column (101) associated with a fractionating column (102) ; at least one duct (125, 127, 131, 132 ; 247, 249, 251, 252, 253) for the supply of the liquid located at the bottom of the purifying-refrigerating column (101) and connecting the latter to the fractionating column (102) by an exchange system (103) ; and at least one reflux duct (118, 119, 120 ; 228, 229) for the distillate from the fractionating column (102) connecting the latter to the purifying-refrigerating column (101) via the said exchange system, characterized in that it also includes :

- at least one duct (137, 138, 120 ; 244, 245, 246) for recirculating a portion of the compressed residual gas, which duct passes through the said exchange system (103) and is connected to the top (101a) of the purifying-refrigerating column,

- at least one duct (124 ; 220, 221) allowing the injection of the distillate (102a) from the fractionating column, directly and without passing through the exchange system (103), into the bottom (101b) of the purifying-refrigerating column (101), and

- at least one duct (125, 127, 129 ; 247, 249, 250) for the injection of the liquid from the purifying-refrigerating column, connecting, directly and without passing through the exchange system, the bottom (101b) of the said purifying-refrigerating column to the top (102a) of the fractionating column (102).

10. An apparatus according to claim 9, characterized in that the gas feed (106) condensed at least partially (108) in the exchange system (103) is conveyed (110) to a separator (109) provided at its bottom with a duct (111) connected directly and preferably to the middle of the fractionating column (102) so as to inject therein the separated liquid phase, and at its top, with a duct (112) for the separated gaseous phase (113) which flows through the said exchange system (103), and which is followed by a duct (114) connected to the purifying-refrigerating column to inject therein the said gaseous phase partially condensed, valve means (V1, V2) being provided to bypass the said separator (Figure 1).

11. An apparatus according to claim 9 or 10, characterized in that to the said exchange system (103) are integrated two closed and independent refrigerating circuits (105, 104) operating at a single pressure level and at one or several pressure levels, respectively (Figure 1).

12. An apparatus according to claim 9, characterized by a gas feed deriving duct (205, 205a) connected to a reboiling system (206) associated with the fractionating column (102), the said deriving duct joining a duct (203, 204a) through which the remainder of the gas feed passes, the junction point (208) of the said ducts communicating with a separator (207) whose bottom is provided with an outflow path (210) for the liquid fraction, the said outflow path dividing into two paths, one (211) of which is connected to the said exchange system (103) and the other (212) is directly connected to the purifying-refrigerating column (101), whereas the top of the separator (207) is connected through a duct (213) to the inlet of a turbine-expander (214) whose outlet is connected to one (211) of the said flow paths to thus form a mixture which is injected through a duct (217) into the purifying-refrigerating column (101) to which it is connected (Figure 2).

13. An apparatus according to claim 12, characterized in that a refrigerating circuit, e. g. of the type with several pressure levels is associated with the said apparatus.

14. An apparatus according to one of claims 9 to 13, characterized in that the said exchange system is constituted by at least one plate exchanger in which the descending fluids cool down and the rising fluids heat up.

15. An apparatus according to one of claims 9 to 14, characterized by a reboiler (159, 160 ; 218, 219) associated with the purifying-refrigerating column (101) and forming part of the exchange system (103).

16. An apparatus according to one of claims 9 to 15, characterized in that the switching from the $C_3+$ cut extracting mode of operation to the $C_2+$ cut extracting mode of operation is performed by means of manually controlled or powered valves (V1 to V20) mounted on the aforesaid ducts.

**Patentansprüche**

1. Verfahren zur Fraktionierung von gasförmigen Massen, wobei entweder Verbindungen, die schwerer als Methan sind ($C_2+$ Fraktion), oder Verbindungen, die schwerer als Ethan sind ($C_3+$ Fraktion), extrahiert werden können, und in dem man die besagte $C_3+$ Fraktion extrahiert durch Einspeisung der teilweise kondensierten, gasförmigen Masse in den Sumpf einer Kolonne zur Reinigung und Kühlung durch Kontakt (101), wobei die besagte Kolonne Restgas als Kopfprodukt erzeugt und als Sumpfprodukt eine Flüssigkeit erzeugt, welche in eine Fraktionierungskolonne (102) eingespeist wird, um als Sumpfprodukt dieser Kolonne eine, die besagte $C_3+$ Fraktion enthaltende Flüssigkeit zu erhalten und als Kopfprodukt ein gasförmiges Destillat zu erhalten, das kondensiert und als Kopfzufuhr in die Kolonne zur Reinigung und Kühlung eingespeist wird, dadurch gekennzeichnet, daß die Extraktion der besagten $C_3+$ Fraktion mit der Extraktion der $C_2+$ Fraktion selektiv austauschbar ist, die

- durch Rückführung eines Teils des verdichteten Restgases (137, 244), das in einem Austauschsystem (103) kondensiert ist (138, 245) und dann in den Kopf der Kolonne zur Reinigung und Kühlung (101) eingespeist ist (120, 246),
- durch direkte Einspeisung (124 ; 220, 221) des aus der Fraktionierungskolonne (102) kommenden gasförmigen Destillats in den Sumpf der Kolonne zur Reinigung und Kühlung (101),
- und durch direkte Einspeisung (125, 127, 129 ; 247, 249, 250) der aus dem Sumpf der Kolonne zur Reinigung und Kühlung (101) kommenden Flüssigkeit in den Kopf der Fraktionierungskolonne (102), durchgeführt werden kann.

2. Verfahren nach Anspruch 1, dadurch gekennzeichnet, daß, wenn man die $C_2+$ Fraktion extrahieren will, die gasförmige Masse (106), nachdem sie wenigstens teilweise im Austauschsystem (103) kondensiert worden ist (108), einerseits in eine, in die Mitte der Fraktionierungskolonne (102) eingespeiste, flüssige Phase (111) getrennt ist (109), und andererseits in eine gasförmige Phase (112) getrennt ist, welche wenigstens teilweise im besagten Austauschsystem kondensiert ist (113) und dann in die Kolonne zur Reinigung und Kühlung (101) eingespeist ist (114), während, bei der Extraktion der $C_3+$ Fraktion, die besagte, im gekühlten Austauschsystem teilweise kondensierte (108, 113), gasförmige Masse (106) in die Kolonne zur Reinigung und Kühlung direkt und ohne Trennen eingespeist ist (114) (Figur 1).

3. Verfahren nach Anspruch 1 oder 2, dadurch gekennzeichnet, daß zusätzliche Kälte dem besagten Austauschsystem zugeführt wird anhand eines Kühlungssystems, das, zur Extraktion der $C_3+$ Fraktion, mit einem einzigen Kältekreislauf (105) mit mehreren Druckpegeln arbeitet, und das, zur Extraktion der $C_2+$ Fraktion, mit zwei verschiedenen Kältekreisläufen, nämlich dem besagten einzigen Kältekreislauf (105) und einem zweiten Kältekreislauf (104) mit einem einzigen Druckpegel arbeitet.

4. Verfahren nach Anspruch 3, dadurch gekennzeichnet, daß, im besagten einzigen Kältekreislauf (105), der zur Extraktion der $C_3+$ Fraktion oder der $C_2+$ Fraktion arbeitet, ein Fluid, bestehend aus einer Mischung von leichten Kohlenwasserstoffen wie Äthan oder Propan, mit einem Molekulargewicht zwischen 30 und 40, oder aus einem reinen Stoff wie Propan, vorzugsweise benutzt wird.

5. Verfahren gemäß Anspruch 3, dadurch gekennzeichnet, daß im besagten zweiten Kältekreislauf (104), der zur Extraktion der $C_2+$ Fraktion arbeitet, ein Fluid bestehend aus einer Mischung von leichten Kohlenwasserstoffen wie Methan, Äthylen, Äthan oder Propan, mit einem Molekulargewicht zwischen 26 und 36, oder aus einem reinen Stoff wie Äthylen oder Äthan benutzt wird.

6. Verfahren nach Anspruch 1, dadurch gekennzeichnet, daß, wenn man die $C_2+$ Fraktion extrahieren will, man einen Teil der gasförmigen Masse (200, 202) umleitet (205, 205a), um das Wiederaufkochen (206) der Fraktionierungskolonne (102) zu gewährleisten, man die umgeleitete gasförmige Masse mit dem verbleibenden Teil (203) der vorher im Austauschsystem (103) teilweise kondensierten gasförmigen Masse (204) mischt (208), man die erhaltene Mischung in eine flüssige Phase und in eine gasförmige Phase trennt (207), und man die flüssige Phase im Austauschsystem (103) weiterkühlt (211), während die gasförmige Phase (213) entspannt (214) und dann mit der besagten weitergekühlten flüssigen Phase (211) gemischt ist, zur Bildung einer gemischten Phase, die man in die Kolonne zur Reinigung und Kühlung (101) unter dem oberen Füllstoffbett ($L_1$) einspeist (217), während, wenn man die $C_3+$ Fraktion extrahieren will, die gasförmige Masse (200) nicht umgeleitet, sondern direkt im Austauschsystem (103) teilweise kondensiert ist (204), wonach sie der besagten Trennung (207) unterworfen ist, zur Bildung einer gasförmigen Phase (213), die, nach der besagten Entspannung (214), in die Kolonne (101) unter dem oberen Füllstoffbett direkt eingespeist ist (215, 217), und einer flüssigen Phase (210, 212), die in die Kolonne zur Reinigung und Kühlung (101) unter dem mittleren Füllstoffbett ($L_2$) direkt eingespeist ist (Figur 2).

7. Verfahren nach Anspruch 6, dadurch gekennzeichnet, daß, wenn zusätzliche Kälte benötigt ist, eine Kühlung durch einen, z. B. mehrere Druckpegel aufweisenden Kältekreislauf, mit der besagten Entspannung (214) gemeinsam durchgeführt ist.

8. Verfahren nach einem der Ansprüche 1 bis 7, dadurch gekennzeichnet, daß, bei irgendeiner der ($C_3+$ oder $C_2+$) zu extrahierenden Fraktion, die besagte Fraktionierungskolonne (102) bei einem Druck arbeitet, der größer ist als derjenige der Kolonne zur Reinigung und Kühlung (101), um die Einspeisung des aus der Fraktionierungskolonne kommenden kondensierten oder nicht kondensierten Destillats in die Kolonne zur Reinigung und Kühlung zu gewährleisten.

9. Vorrichtung zur Durchführung des Verfahrens nach einem der vorangehenden Ansprüche, und der Gattung, die eine Kolonne zur Reinigung und Kühlung durch Kontakt (101), die mit einer Fraktionierungskolonne (102) verbunden ist ; wenigstens eine Leitung (125, 127, 131, 132, ; 247, 249, 251, 252, 253) zur Einspeisung der sich im Sumpf der Kolonne zur Reinigung und Kühlung (101) befindenden Flüssigkeit, und die diese Kolonne mit der Fraktionierungskolonne (102) über ein Austauschsystem (103) verbindet ; und wenigstens eine Rückflußleitung (118, 119, 120 ; 228, 229) für das Destillat der Fraktionierungskolonne (102), die diese Kolonne mit der Kolonne zur Reinigung und Kühlung (101) über das besagte Austauschsystem verbindet, aufweist, dadurch gekennzeichnet, daß sie außerdem :
- wenigstens eine Leitung (137, 138, 120 ; 244, 245, 246) zum Wiederumlauf eines Teils des verdichteten Restgases, welche Leitung das besagte Austauschsystem (103) durchquert und mit dem Kopf (101a) der Kolonne zur Reinigung und Kühlung in Verbindung steht,
- wenigstens eine Leitung (124 ; 220, 221), die die Einspeisung des Destillats (102a) der Fraktionierungskolonne in den Sumpf (101b) der Kolonne zur Reinigung und Kühlung (101), direkt und ohne das Austauschsystem zu durchqueren, ermöglicht, und
- wenigstens eine Leitung (125, 127, 129 ; 247, 249, 250) zur Einspeisung der Flüssigkeit aus der Kolonne zur Reinigung und Kühlung, die den Sumpf (101b) dieser Kolonne mit dem Kopf (102a) der Fraktionierungskolonne (102) direkt und ohne das Austauschsystem zu durchqueren, verbindet, aufweist.

10. Vorrichtung nach Anspruch 9, dadurch gekennzeichnet, daß die im Austauschsystem (103) teilweise kondensierte (108) gasförmige Masse (106) in einen Abscheider (109) eingeleitet ist (110), der am Unterteil eine, mit der Fraktionierungskolonne (102) direkt und vorzugsweise mit der Mitte derselben verbundene Leitung (111) aufweist, um die getrennte flüssige Phase in diese Kolonne einzuspeisen, und der am Kopf eine Leitung (112) für die getrennte gasförmige Phase (113) aufweist, die das besagte Austauschsystem (103) durchquert, und die von einer, mit der Kolonne zur Reinigung und Kühlung verbundenen Leitung (114) verlängert ist, zur Einspeisung der besagten, teilweise kondensierten gasförmigen Phase in diese Kolonne, während ein Mittel, wie Ventile (V1, V2), zum Umgehen des besagten Abscheiders vorgesehen ist (Figur 1).

11. Vorrichtung gemäß Anspruch 9 oder 10, dadurch gekennzeichnet, daß zwei geschlossene und unabhängige Kühlkreisläufe (105, 104), die mit einem einzigen Druckpegel bzw. mit einem oder mehreren Druckpegeln arbeiten, zum besagten Austauschsystem (103) integriert sind (Figur 1).

12. Vorrichtung nach Anspruch 9, gekennzeichnet durch eine Umleitung (205, 205a) für die gasförmige Masse, die mit einem, mit der Fraktionierungskolonne (102) verbundenen Wiederaufkochsystem (206) verbunden ist, und die mit einer, von dem verbleibenden Teil der gasförmigen Masse durchquerten Leitung (203, 204a) vereinigt ist, wobei die Vereinigung (208) der besagten Leitungen mit einem Abscheider (207) in Verbindung steht, dessen Unterteil eine Leitung (210) zur Strömung der flüssigen Fraktion aufweist, die in zwei Leitungen geteilt ist, deren eine (211) mit dem besagten Austauschsystem (103) verbunden ist, und deren andere (212) mit der Kolonne zur Reinigung und Kühlung (101) direkt verbunden ist, während der Kopf des Abscheiders (207) durch eine Leitung (213) mit dem Einlaß eines Turbo-Expanders (214) verbunden ist, dessen Auslaß mit einer (211) der besagten Strömungsleitungen verbunden ist, um eine Mischung zu bilden, die durch eine Leitung (217) in die Kolonne zur Reinigung und Kühlung (101), mit welcher diese Leitung verbunden ist, eingespeist ist (Figur 2).

13. Vorrichtung nach Anspruch 12, dadurch gekennzeichnet, daß ein Kühlungskreislauf, z. B. mit mehreren Druckpegeln, mit der besagten Vorrichtung in Verbindung steht.

14. Vorrichtung nach einem der Ansprüche 9 bis 13, dadurch gekennzeichnet, daß das besagte Austauschsystem aus wenigstens einem Platten-Austauscher besteht, in dem die sich abkühlenden Fluide nach unten fließen und die sich aufwärmenden Fluide nach oben fließen.

15. Vorrichtung nach einem der Ansprüche 9 bis 14, gekennzeichnet durch einen Wiederaufkocher (159, 160 ; 218, 219), der mit der Kolonne zur Reinigung und Kühlung (101) in Verbindung steht und der zum Austauschsystem (103) gehört.

16. Vorrichtung nach einem der Ansprüche 9 bis 15, dadurch gekennzeichnet, daß die Umstellung zwischen der Extraktion der $C_3+$ Fraktion und der Extraktion der $C_2+$ Fraktion anhand von, auf den besagten Leitungen angeordneten manuell- oder motorbetätigten Ventilen (V1 bis V20) durchgeführt ist.

0 197 808

FIG. 1

_Fig: 2_